# EUROPEAN PATENT APPLICATION

(11) **EP 2 095 839 A1**
(43) Date of publication of application: **02.09.2009**
(21) Application number: 09153917.1
(22) Date of filing: 27.02.2009
(51) Int. Cl.: A61M 5/46, A61B 19/00

(54) **Device and medical instrument for preventing excessive insertion of a rod-like member**

(30) Priority: 29.02.2008 JP 2008049107
(71) Applicant: Tyco Healthcare Group LP, Mansfield, MA 02048 (US)
(72) Inventor: Harada, Hisataka, Shizuoka-ken 437-004 (JP)
(74) Representative: Körber, Martin Hans

(57) **Abstract**

A device (30) for preventing excessive insertion of a rod-like member (20) when the rod-like member is inserted into a gastrostomy catheter (10), and to a medical instrument provided with the same. The device comprises a reference position establishment part (31) which can be installed at a base end opening side of the gastrostomy catheter (10), a mobile part (32) which can be fixed to the endoscope (20), and a linking part (33). The linking part (33) can change the gap between the reference position establishment part (31) and the mobile part (32) within a range no greater than a set length, and links the reference position establishment part (31) and the mobile part (32). Furthermore, a bending instrument (40) for bending the tip end of the endoscope (20) when said tip end portion projects a prescribed length outwards from a through-hole of a stomach-internal fixed part is fitted to the tip end of the endoscope (20).

## Description

### FIELD OF THE INVENTION

The present invention relates to a device for preventing excessive insertion which is used when a rod-like member is inserted into a gastrostomy catheter which is positioned in a fistula formed in a patient's body, and to a medical instrument provided with the same.

### BACKGROUND OF THE INVENTION

It is conventional practice to position a gastrostomy catheter in a fistula formed in a patient's body, and to insert an endoscope into the gastrostomy catheter to observe the inside of the stomach as well as to confirm the position of the gastrostomy catheter. In such cases, it is necessary to change the orientation of the tip end of the endoscope so as to be able to see in various directions in order to accurately confirm the state of the inside of the stomach and position of the gastrostomy catheter. Consequently, a bending instrument or the like which can change the orientation of the tip end of the endoscope is used when the endoscope is inserted a prescribed length into the gastrostomy catheter. In such cases, if more than is necessary of the endoscope is inserted into the stomach from the gastrostomy catheter, the bent portion of the endoscope grows larger, making it impossible to make accurate observations. Additionally, the endoscope may be damaged.

In order to prevent this, it has been envisaged to prevent the endoscope from being inserted beyond a prescribed length into the gastrostomy catheter using a stopper, or the like (see Japanese Unexamined Utility Model Registration Application H1-130317, for example). The laser probe disclosed in H1-130317 consists of a probe main body in which an optical fiber serves as a light guide. The probe main body has a configuration in which it is inserted into the endoscope for use. A stopper or the like is then fixed to a prescribed portion of the probe main body, and it is possible to regulate the probe main body by means of the stopper so that it is not inserted more than necessary into the endoscope.

However, with the laser probe mentioned above, when the probe main body is inserted into endoscopes of different lengths, the length of the tip end portion of the probe main body which projects from the tip end of the endoscope may be too great or too small. Consequently, when the laser probe is inserted into the stomach via the gastrostomy catheter, the tip end of the laser probe cannot be inserted into the stomach to a suitable length in accordance with the length of the gastrostomy catheter. Furthermore, it is not just the endoscope which is inserted into the gastrostomy catheter, but also a push-rod, for example, for making the stomach-internal fixed part extend in an elongate fashion when the gastrostomy catheter is positioned in the fistula formed in the body of the patient. Here too it is necessary to insert the push-rod to a suitable length in accordance with the length of the gastrostomy catheter.

### SUMMARY OF THE INVENTION

In one aspect of the present invention, a device is used for preventing excessive insertion when a rod-like member is inserted into a gastrostomy catheter comprising a tubular part and a stomach-internal fixed part. The fixed part comprises an expanded body which links with a tip end of the tubular part. The tip end is provided with a through-hole, and said fixed part extends in an elongate fashion due to a portion on a through-hole side being pushed in the direction of a tip end side. The device generally comprises a reference position establishment part which can be installed at a prescribed portion on a base end opening side of the gastrostomy catheter in a state in which it can move in a longitudinal direction of the rod-like member. A mobile part can be fixed to a prescribed portion of the rod-like member. A linking part can change a gap between the reference position establishment part and the mobile part within a range which is no greater than a set length, and links the reference position establishment part and the mobile part.

In another aspect of the present invention, a medical instrument in combination with the device described in the preceding paragraph.

Other objects and features will be in part apparent and in part pointed out hereinafter.

### BRIEF DESCRIPTION OF THE DRAWINGS

FIG. 1 shows a gastrostomy catheter, where (a) is a plan view, (b) is a front view, and (c) is a bottom view.

FIG. 2 is a front view showing a state in which the device for preventing excessive insertion according to the first embodiment of the present invention is fitted to an endoscope.

FIG. 3 is a front view showing the endoscope.

FIG. 4 shows the device for preventing excessive insertion according to the first embodiment of the present invention, where (a) is a plan view, (b) is a front view, (c) is a side view, and (d) is a bottom view.

FIG. 5 shows a state in which the mobile part of the device for preventing excessive insertion according to the first embodiment has moved to the reference position establishment part side, where (a) is a front view, and (b) is a side view.

FIG. 6 is an oblique view showing a bending instrument.

FIG. 7 is a fragmentary cutaway in cross section showing a state in which the endoscope, to which the device for preventing excessive insertion according to the first embodiment is fitted, is positioned above the gastrostomy catheter in the patient's body.

FIG. 8 is a fragmentary cutaway in cross section showing a state in which the endoscope, to which the device for preventing excessive insertion according to the first embodiment is fitted, is inserted into the gastrostomy catheter in the patient's body.

FIG. 9 is a fragmentary cutaway in cross section showing a state in which the position of the gastrostomy catheter is confirmed using the endoscope to which the device for preventing excessive insertion according to the first embodiment is fitted.

FIG. 10 shows the device for preventing excessive insertion according to a second embodiment of the present invention, where (a) is a plan view, (b) is a front view, and (c) is a bottom view.

FIG. 11 is a fragmentary cutaway in cross section showing a state in which the endoscope, to which the device for preventing excessive insertion according to the second embodiment is fitted, is inserted in the gastrostomy catheter in the patient's body.

FIG. 12 is a fragmentary cutaway in cross section showing a state in which the position of the gastrostomy catheter is confirmed using the endoscope to which the device for preventing excessive insertion according to the second embodiment is fitted.

FIG. 13 is a front view showing the device for preventing excessive insertion according to a third embodiment of the present invention.

FIG. 14 is a front view showing a state in which the linking part of the device for preventing excessive insertion according to the third embodiment of the present invention is in a contracted state.

FIG. 15 is a fragmentary cutaway in cross section showing a state in which the endoscope, to which the device for preventing excessive insertion according to the third embodiment is fitted, is inserted in the gastrostomy catheter in the patient's body.

FIG. 16 is a fragmentary cutaway in cross section showing a state in which the position of the gastrostomy catheter is confirmed using the endoscope to which the device for preventing excessive insertion according to the third embodiment is fitted.

FIG. 17 shows the device for preventing excessive insertion according to a fourth embodiment of the present invention, where (a) is a plan view, (b) is a front view, and (c) is a bottom view.

FIG. 18 is an oblique view showing the fitting instrument which is used according to the fourth embodiment of the present invention.

FIG. 19 is a front view showing a state in which the push-rod of the fitting instrument to which the device for preventing excessive insertion according to the fourth embodiment is attached is inserted in the gastrostomy catheter.

FIG. 20 is a front view showing a state in which the stomach-internal fixed part of the gastrostomy catheter is extended using the fitting instrument to which the device for preventing excessive insertion according to the fourth embodiment is attached.

FIG. 21 is a fragmentary cutaway in cross section showing a state in which the stomach-internal fixed part of the gastrostomy catheter has entered the stomach by means of the fitting instrument to which the device for preventing excessive insertion according to the fourth embodiment is attached.

Corresponding reference characters indicate corresponding parts throughout the drawings.

### DESCRIPTION OF THE PREFERRED EMBODIMENTS

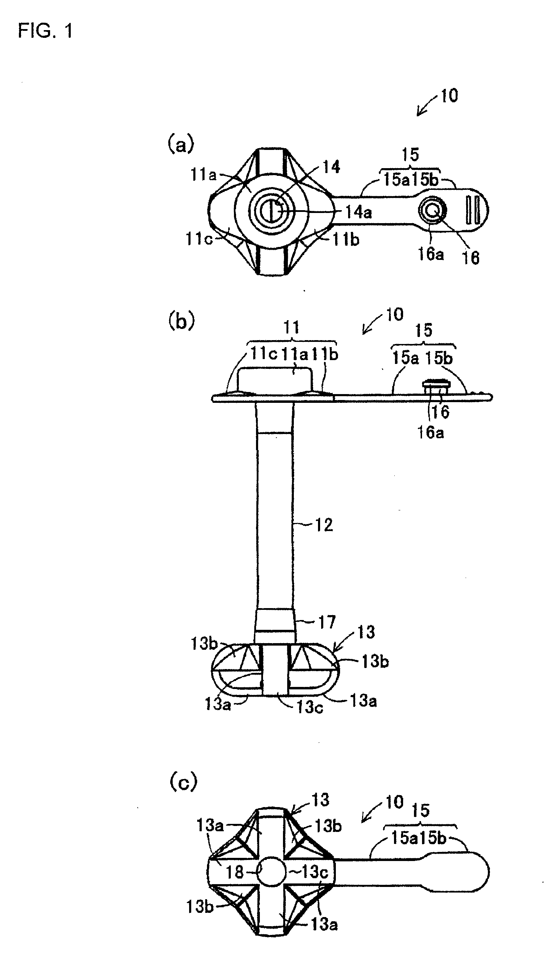

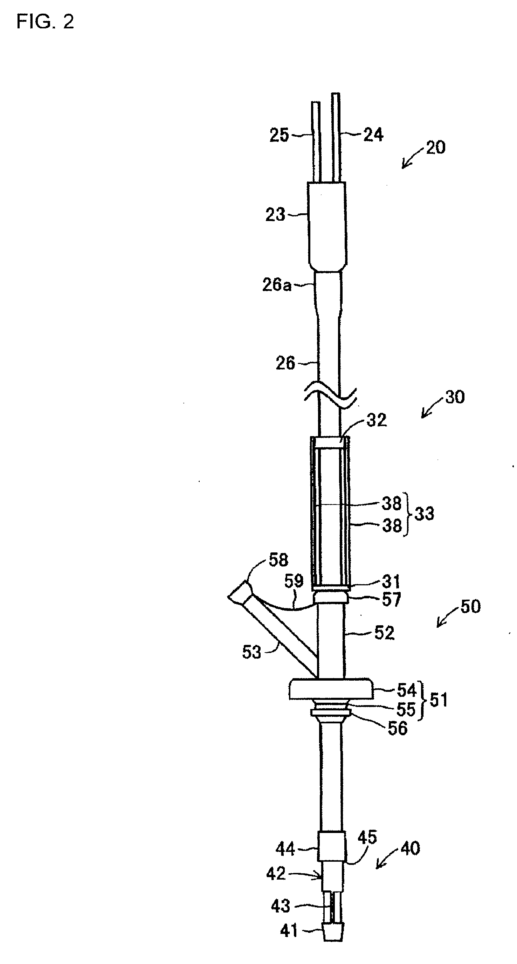

A first embodiment of the present invention is described below using the figures. Figure 1 shows a gastrostomy catheter 10 pertaining to the first embodiment. Figure 2 shows a state in which a device 30 for preventing excessive insertion, a bending instrument 40, and an insertion aid 50 etc. are fitted to an endoscope 20 which acts as the rod-like member according to the present invention which is inserted into the gastrostomy catheter 10. The medical instrument according to the first embodiment comprises the gastrostomy catheter 10, endoscope 20, device 30 for preventing excessive insertion, bending instrument 40 and insertion aid 50, among other things. The gastrostomy catheter 10 consists of an external fixed part 11, a tubular part 12 which is linked to the centre of the lower surface of the external fixed part 11, and a stomach-internal fixed part 13 which is attached to the lower end of the tubular part 12. All the components are made of a soft plastic material such as polyurethane or silicone.

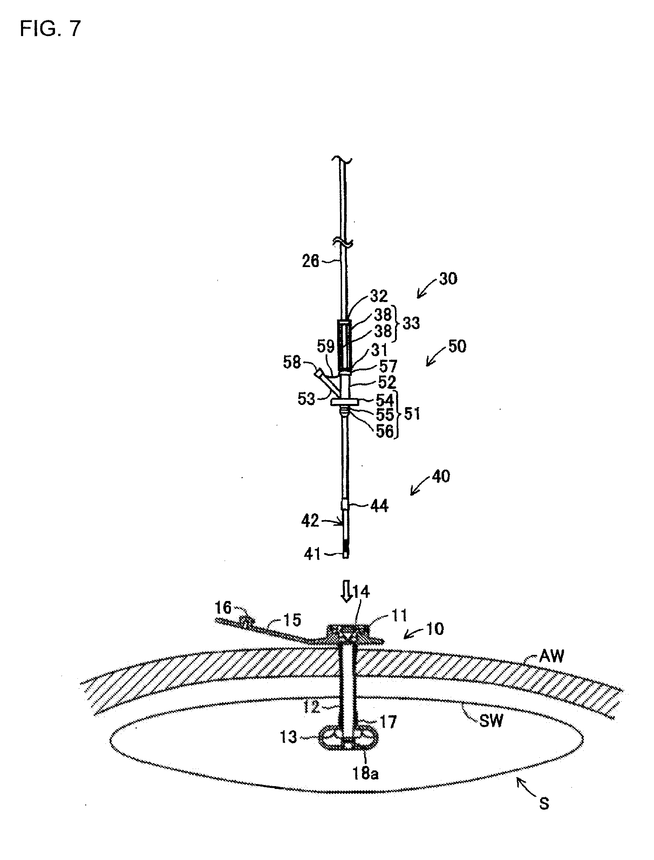

In the description that follows, the external fixed part 11 will be referred to as the upper side, and the stomach-internal fixed part 13 will be referred to as the lower side. The external fixed part 11 comprises an insertion opening 11a which is annular and fairly thick, and projecting pieces 11b, 11c which project at both sides from the lower end of both side parts of the insertion opening 11a. The outline of the portion including the projecting pieces 11b, 11c and the insertion opening 11a is elliptical, when seen as a plane, and the function of these projecting pieces 11b, 11c is to prevent the gastrostomy catheter 10 from being pulled into the stomach S (see FIGs. 7 to 9). A valve body 14a which is formed with a central slit is then provided on the inner peripheral surface of an insertion hole 14 which is formed in the centre of the insertion opening 11a, passing through vertically. Furthermore, an engagement groove is formed along the circumference at the upper side of the valve body 14a on the inner peripheral surface of the insertion hole 14, although this is not depicted.

A cover part 15 for closing off the insertion hole 14 of the insertion opening 11a is then joined to the tip end of the projecting piece 11b. The cover part 15 comprises an elongate strip-shaped linking part 15a which is linked to the end of the projecting piece 11b, and a broad part 15b which is shorter and wider than the strip-shaped linking part 15a, and is formed at the tip end of the strip-shaped linking part 15a. A stopper part 16 shaped like a column which is short in the axial direction is then provided on the broad part 15b. The strip-shaped linking part 15a is flexible, and it can flex so as to vertically rotate, or bend at a sharp angle, with the linking part to the projecting piece 11b at the centre. The stopper part 16 is provided on the strip-shaped linking part 15a side portion of the broad part 15b, so as to face the insertion hole 14 when the strip-shaped linking part 15a is bent to position the broad part 15b above the insertion opening 11a.

The stopper part 16 is formed with a columnar shape which can fit into the insertion hole 14, and it is provided on its outer peripheral surface with an annular projection 16a running along its periphery, the projection being able to detachably engage with the engagement groove formed on the inner peripheral surface of the insertion hole 14. Accordingly, it is possible to engage the engagement groove with the annular projection 16a by bending the strip-shaped linking part 15a so that it is upwardly inverted, and pushing the stopper part 16 into the insertion hole 14, and this makes it possible to close off the insertion hole 14 of the insertion opening 11a in an airtight and liquid-tight manner. It is also possible to open the insertion hole 14 of the insertion opening 11a by pulling the broad part 15b to release the fitting between the stopper part 16 and the insertion hole 14.

The tubular part 12 is formed as a cylindrical shape, and a through-orifice (not depicted) for allowing the passage of fluids such as nutrients and food in fluid form is formed inside it; the upper end of the through-orifice links in communication with the insertion hole 14 of the external fixed part 11. The stomach-internal fixed part 13 is connected to the tubular part 12 via a connection part 17 which is fixed to the lower end of the tubular part 12. The connection part 17 is formed as a cylinder for covering the outer peripheral surface of the tubular part 12 and is integrally formed with the stomach-internal fixed part 13. The connection part 17 is fixed to the lower end of the tubular part 12, in a state in which it cannot be removed from the tubular part 12.

The stomach-internal fixed part 13 comprises four strip-shaped linking parts 13a which are linked to the edge of a lower end opening of the connection part 17 and extend in four directions, four linking film parts 13b which are provided between the upper parts of each of the linking parts 13a and form a substantially dome-shaped stomach wall contact part with the four linking parts 13a, and a converging part 13c where the tip ends of all of the linking parts 13a converge. The four linking parts 13a comprise strip-shaped members which are bent into substantially semi-circular shapes which split into four directions from the lower end of the connection part 17, respectively extending downwards from the horizontal, after which they converge below the central axis of the tubular part 12, linking to form the converging part 13c. In other words, the converging part 13c allows each of the linking parts 13a to link by joining the lower ends of all of the linking parts 13a, and it is also positioned by all of the linking parts 13a below the central axis of the tubular part 12.

Moreover, the stomach-internal fixed part 13 which comprises the linking parts 13a, linking film parts 13b and the converging part 13c is integrally formed together with the connection part 17. Furthermore, all of the linking parts 13a and linking film parts 13b are made of a soft, flexible, elastic material, and the overall flat, substantially spherical shape is normally maintained by means of its elasticity, as shown in FIG. 1. The linking parts 13a and linking film parts 13b can then be extended to make them straight and elongate by pulling the converging part 13c downwards. Furthermore, the lower end of the through-orifice of the tubular part 12 opens between the upper ends of the linking parts 13a.

Spaces formed between the lower parts of each of the linking parts 13a then form channels for the passage of fluids such as nutrients and food in fluid form sent out from the through-hole of the tubular part 12 into the stomach S. A through-hole 18 is additionally formed in the centre of the converging part 13c, and a cylindrical engagement part 18a (see FIGs. 7 to 9) is formed at the upper part of the through-hole 18 (converging part 13c). Hence, the through-hole 18 is configured by the inner peripheral surface of the cylindrical engagement part 18a, and its diameter is smaller than the diameter of the insertion hole 14 of the external fixed part 11 and the diameter of the through-orifice of the tubular part 12. The stomach-internal fixed part 13 configured in this manner is positioned on the inner surface of the patient's stomach wall SW (see FIGs. 7 to 9) and its function is to prevent the gastrostomy catheter 10 from being removed from the patient's body.

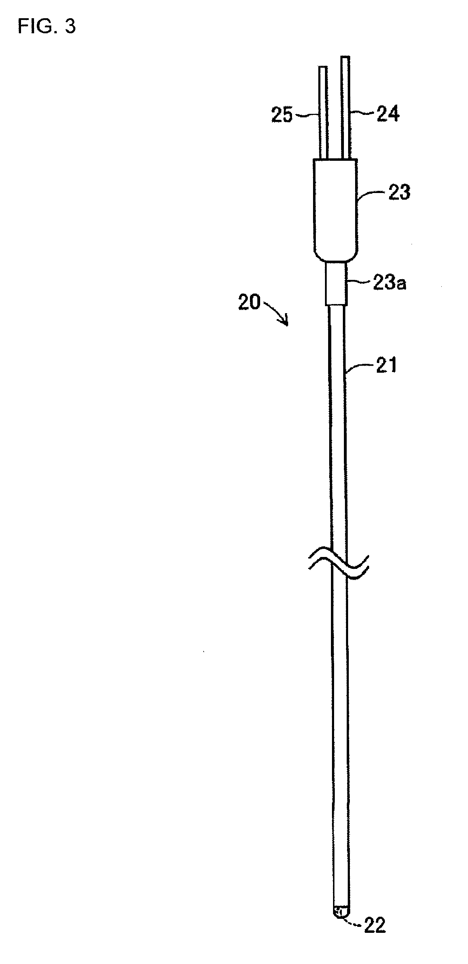

As shown in FIG. 3, the endoscope 20 has a configuration in which a lens 22 is attached to the tip end of a shaft 21, and a connection part 23 is attached to the rear end thereof. The shaft 21 consists of a bundle of fibres comprising a plurality of light guides (not depicted) for irradiating light onto the stomach wall SW, and an image guide (not depicted) for sending reflected light via the lens, and it is flexible. The connection part 23 is linked to wiring 24 which connects the image guide to an image display device (not depicted), and to wiring 25 which connects the light guides to a light source device (not depicted). The lens 22 sends images obtained by the irradiated light to the image display device via the image guide and the wiring 24.

Hence, the light guides irradiate the inner surface of the stomach wall SW with light sent from the light source device to make observation possible, and the image guide sends the light which is reflected from the inner surface of the stomach wall SW and focused by means of the lens 22 to the image display device. The image display device then enlarges the images based on the reflected light sent and displays them on an image display part provided in the image display device. Furthermore, as shown in FIG. 2, the shaft 21 is covered by a sheath 26, and it is possible to prevent soiling of the shaft by covering it with the sheath 26. The sheath 26 consists of a tube in which the tip end is closed off by a light-transmissive window part (not depicted), and in which the diameter of the base end 26a on the opening side is somewhat larger than the other portion, and it is flexible.

The sheath 26 is formed with a thickness such that it can cover the shaft 21 in a state in which it is in close contact with the outer peripheral surface of the shaft 21, and it is attached to the shaft 21 by pushing the tip-end narrow-diameter part 23a of the connection part 23 into the base end 26a. The sheath 26 is prevented from being removed from the shaft 21 using a member such as a clamp, a fastener, a clasp or the like. When assembled in this state, a configuration is adopted so that the lens 22 is in contact with the inner surface of the window part. Furthermore, the surface of the window part forms a curving protrusion which projects downwards.

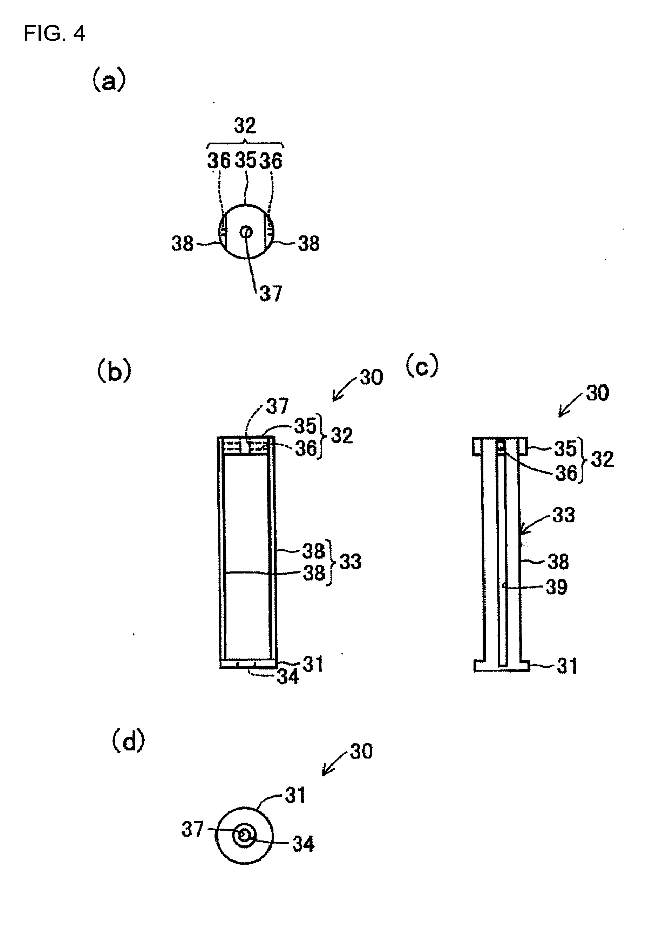

As shown in FIG. 4, the device 30 for preventing excessive insertion consists of a reference position establishment part 31, a mobile part 32 and a linking part 33. The reference position establishment part 31 consists of an annular body in the centre of the disc of which is formed a through-hole 34 through which the shaft 21 which is covered by the sheath 26 can pass with clearance. The mobile part 32 consists of a mobile part main body 35 which is formed with plane parts in which both the left- and right-hand portions of the disc (the left- and right-hand side portions in the states shown in FIGs. 4(a) and (b)) have been cut away, these plane parts running parallel to each other on both the left- and right-hand sides, and guide screws 36 which are each attached to the plane parts of the mobile part main body 35.

Furthermore, the diameter at the centre of the mobile part main body 35 is smaller than the through-hole 34, but a through-hole 37 through which the shaft 21 covered by the sheath 26 can pass is formed therein. Screw holes are formed from the centre of both plane parts of the mobile part main body 35 towards the inner peripheral surface of the through-hole 37, and the guide screws 36 screw into these screw holes. When one end of the guide screws 36 is positioned on the inner peripheral surface of the through-hole 37, the other end of the guide screws reaches a state in which it protrudes outwards from the plane parts, and one end of the guide screws 36 can be made to project into the through-hole 37 by turning the guide screws 36 in one direction.

The linking part 33 is integrally formed with the reference position establishment part 31, and consists of a pair of rail-like guiding pieces 38 which run vertically. The outline of the guide pieces 38 has a shape seen from above in which both arcuate end parts are joined by a straight line, and seen from the front they are arranged laterally symmetrically, comprising members formed as a longitudinal frame with a gap between them. Hence, when the mobile part main body 35 has been positioned between the pair of guide pieces 38 in a state in which the plane parts of the mobile main body 35 and the plane parts of the guide pieces 38 are aligned, the pair of guide pieces 38 is formed so that the outline is circular when seen from above. Consequently, the mobile part main body 35 can slide vertically between the pair of guide pieces 38 in a state in which the axial rotation thereof is controlled.

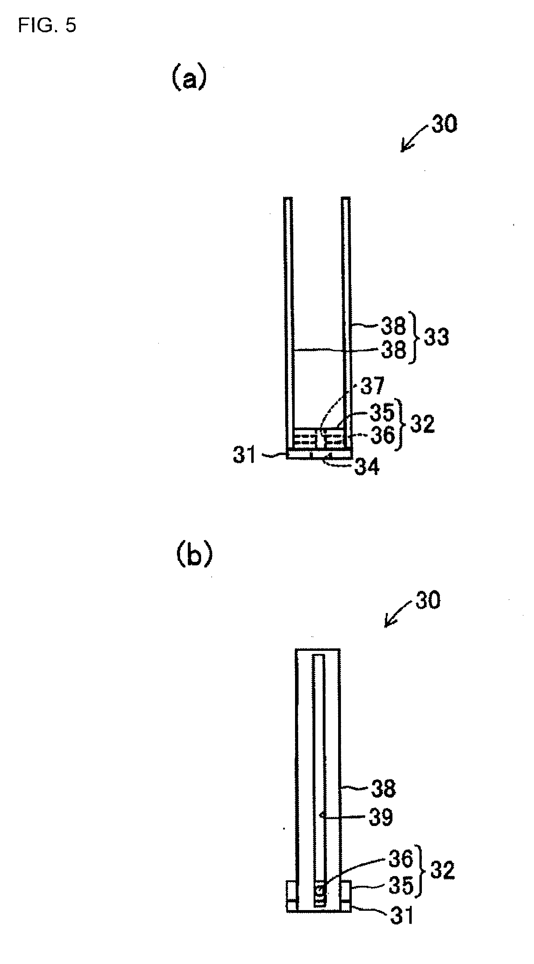

Furthermore, opposing guide holes 39 are formed in the centre of the two guide pieces 38, running vertically. These guide holes 39 have a width enabling the guide screws 36 of the mobile part 32 to be positioned inside in a mobile fashion. The mobile part 32 is arranged between the pair of guide pieces 38 in a state in which the two guide screws 36 are positioned inside the guide holes 39 of the respectively opposing guide pieces 38, and the mobile part is prevented from being removed from the linking part 33 by the engagement of the guide screws 36 with the peripheral edge of the guide holes 39. Figure 5 shows a state in which the mobile part 32 has moved to the lower end of the linking part 33, and the mobile part 32 can be moved between the position shown in FIGs. 4(b) and (c) and the position shown in FIG. 5. The maximum length which the mobile part 32 can move along the guide holes 39 is the set length pertaining to the present invention.

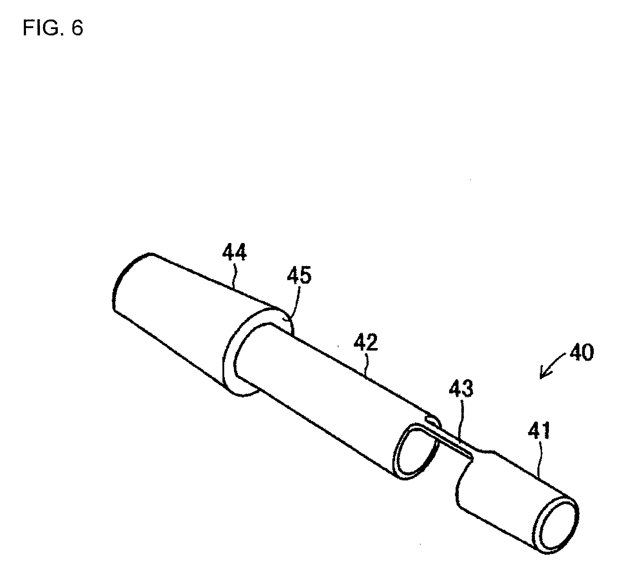

The bending instrument 40 is used to bend the tip end portion of the shaft 21 of the endoscope 20 so as to change the direction of observation of the endoscope 20. As shown in FIG. 6, the bending instrument 40 consists of a cylindrical fixed part 41, a cylindrical stepped sliding part 42 which is longer in the axial direction than the fixed part 41, and a linear linking part 43 which links the fixed part 41 and the sliding part 42. The fixed part 41 consists of a cylindrical body in which the diameter of the lower portion tapers to become somewhat smaller than the diameter of the upper portion. The inner diameter of the upper portion of the fixed part 41 is somewhat greater than the outer diameter of the sheath 26, and the inner diameter of the lower end part of the fixed part 41 is somewhat smaller than the outer diameter of the sheath 26. Furthermore, the outer diameter of the upper portion of the fixed part 41 is smaller than the diameter of the through-hole 18 of the stomach-internal fixed part 13.

Consequently, the fixed part 41 can penetrate into the gastrostomy catheter 10 from the insertion hole 14 towards the through-hole 18. Furthermore, when the sheath 26 is inserted into the fixed part 41 from the upper part towards the lower part from the tip end, the outer peripheral part of the tip end of the sheath 26 abuts the inner peripheral part at the lower end of the fixed part 41. Consequently, the sheath 26 cannot pass through inside the fixed part 41 and it is in a state in which it covers the shaft 21. Moreover, when the bending instrument 40 is being used, the fixed part 41 is fixed to the outer peripheral part of the tip end of the sheath 26 by means of adhesive.

The inner diameter of the sliding part 42 is somewhat greater than the outer diameter of the sheath 26, and when the sheath 26 passes through the sliding part 42, the sliding part 42 is able to slide along in the length direction of the sheath 26. Furthermore, a latch part 44 which has a greater diameter than the lower portion of the sliding part 42 is formed at the upper part of the sliding part 42. The latch part 44 is formed with a shape which tapers upwards in which the outer diameter at the upper portion is smaller than the outer diameter at the lower portion. A step part 45 having a horizontal surface is then formed at the lower end of the latch part 44.

The outer diameter of the step part 45 is smaller than the diameter of the insertion hole 14 of the external fixed part 11 and of the through-hole of the tubular part 12, but greater than the diameter of the through-hole 18 of the stomach-internal fixed part 13. Consequently, when the bending instrument 40 is inserted into the gastrostomy catheter 10 from the insertion hole 14 towards the through-hole 18, the bending instrument 40 passes through the insertion hole 14 of the external fixed part 11 and the through-hole of the tubular part 12. The lower portion of the sliding part 42 then also passes through the through-hole 18 of the stomach-internal fixed part 13, but when the step part 45 reaches the cylindrical engagement part 18a, the step part 45 and the cylindrical engagement part 18a engage, and the latch part 44 cannot pass through the through-hole 18.

Furthermore, the linear linking part 43 is flexible and links the upper end edge of the fixed part 41 and the portion opposite at the lower end edge of the sliding part 42. After the shaft 21 of the endoscope 20 has passed inside the bending instrument 40 together with the sheath 26, and the outer peripheral part of the tip end of the sheath 26 has engaged with the inner peripheral part at the lower end of the fixed part 41, when the sheath 26 etc. are inserted, the direction in which the tip end of the endoscope 20 is oriented can be changed by bending the linear linking part 43. The linear linking part 43 is positioned on the inner peripheral side when the shaft 21 etc. is bent, and it keeps the distance between the upper end edge of the fixed part 41 and the lower end edge of the sliding part 42 substantially constant.

The insertion aid 50 is attached to the gastrostomy catheter 10 to provide smoother insertion of the endoscope 20 etc. into the gastrostomy catheter 10, and it consists of a connection part 51, insertion opening 52 and an air supply opening 53. The connection part 51 consists of a substantially cylindrical engagement part 55 which is formed in the centre of the lower surface of an annular connection part main body 54, and a through-hole for allowing the insertion of the shaft 21 covered by the sheath 26 is formed therein. Furthermore, the connection part main body 54 is formed with an annular shape which is somewhat smaller than the insertion opening 11a of the gastrostomy catheter 10, and the engagement part 55 is formed with a cylindrical shape having four different levels.

The engagement part 55 is made up of an uppermost level in which the outer peripheral surface which has a larger diameter at its upper part than its lower part has an oblique surface, a second level which has the same diameter as the lower part of the uppermost level, a third level which has substantially the same diameter as the upper part of the uppermost level, and a lowermost level in which the outer peripheral surface which has substantially the same diameter as the second level at its upper part, and a smaller diameter at its lower part than its upper part has an oblique surface. The third level of the engagement part 55 configures an annular projection 56 which is able to detachably engage with the engagement groove formed in the insertion hole 14 of the gastrostomy catheter 10, and when the annular projection 56 engages with the engagement groove, a state of air-tightness and liquid-tightness is achieved between the engagement part 55 and the peripheral surface of the insertion hole 14.

The insertion opening 52 is formed with a cylindrical shape and an insertion hole enabling the insertion of the shaft 21 covered by the sheath 26 is formed therein, an annular reinforcing rib 57 being formed on the edge of the opening at the upper end. Furthermore, the through-hole formed inside the insertion opening 52 and the insertion hole formed inside the connection part 51 have the same diameter and are also coaxially linked in communication. The air supply opening 53 is formed as a cylindrical shape extending obliquely upwards from the side at the lower end of the insertion opening 52 in a state in which it is inclined at approximately 45° to the insertion opening 52, and it is narrower in diameter than the insertion opening 52. An annular reinforcing rib 58 is furthermore formed on the edge of the opening at the upper end of the air supply opening 53.

An air supply device (not depicted) is connected to the reinforcing rib 58 of the air supply opening 53, and air which is supplied from the air supply device passes through inside the air supply opening 53 and is sent to the lower end inside the insertion opening 52. Furthermore, an airflow channel (not depicted) for allowing the passage of air is formed between the lower end inside the insertion opening 52 and the lower end inside the connection part 51; air sent to the lower end inside the insertion opening 52 is released to the outside from the lower end of the connection part 51. A substantially triangular sheet-like reinforcing grip part 59 for strengthening the area between the insertion opening 52 and the air supply opening 53 and also for facilitating holding of the insertion aid 50 with the hand is formed between the insertion opening 52 and the air supply opening 53.

A description of the method of confirming the position of the gastrostomy catheter 10 using the endoscope 20, sheath 26, device 30 for preventing excessive insertion, bending instrument 40 and insertion aid 50 configured in the manner described above will be given next, with reference to FIGs. 7 to 9. Figure 7 shows a state in which the gastrostomy catheter 10 is positioned in a fistula provided in the abdominal wall AW and the stomach wall SW of a patient, where the gastrostomy catheter 10 is positioned in the fistula using an instrument for fitting it which will be described later. In the state shown in FIG. 7, the stopper part 16 of the gastrostomy catheter 10 is removed from the insertion hole 14 to open the upper end of the insertion hole 14. Furthermore, the endoscope 20 to which are fitted the sheath 26, device 30 for preventing excessive insertion, bending instrument 40 and insertion aid 50 is positioned above the gastrostomy catheter 10.

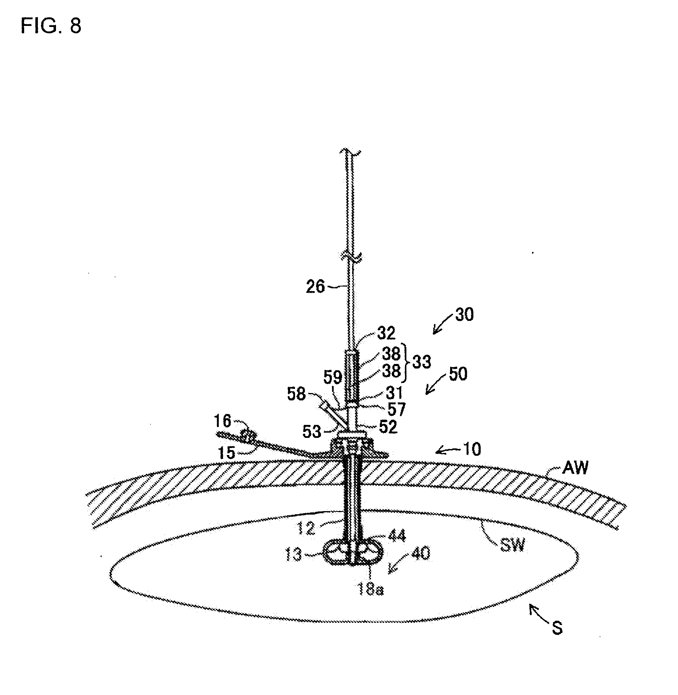

The endoscope 20 and other components which are in this state are moved down in the direction of the arrow in the figures so that the shaft 21 projecting from the lower end of the insertion aid 50 is inserted into the insertion hole 14 of the gastrostomy catheter 10 together with the sheath 26 and bending aid 40. At this time, an operator holds both sides of the insertion opening 11a where the projecting pieces 11b, 11c are not formed on the gastrostomy catheter 10 with one hand, and holds the insertion aid 50 with the other hand, and pushes the insertion aid 50 into the gastrostomy catheter 10. As shown in FIG. 8, this makes it possible to engage the insertion aid 50 with the gastrostomy catheter 10.

The engagement is brought about by the engagement of the engagement part 55 of the insertion aid 50 with the engagement groove of the gastrostomy catheter 10, and a state of air-tightness and liquid-tightness is achieved between the insertion aid 50 and the gastrostomy catheter 10. In this state, the endoscope 20 is then further inserted towards the lower side of the gastrostomy catheter 10 together with the sheath 26 and bending instrument 40. This operation is carried out while the operator is looking at the image display part of the image display device, and insertion of the endoscope 20 and other members is stopped when the tip end of the endoscope 20 has reached the cylindrical engagement part 18a, and the device 30 for preventing excessive insertion is fixed to the shaft 21 with the sheath 26 interposed.

The mobile part 32 is positioned at the upper end of the guide holes 39 of the linking part 33, in a state in which the reference position establishment part of the device 30 for preventing excessive insertion is in abutment with the reinforcing rib 57 of the insertion aid 50. The guide screws 36 are then turned so that the shaft 21 which is positioned inside the mobile part main body 35 is pressured, via the sheath 26, at its tip end. By means of this, the mobile part 32 is fixed to the shaft 21. Moreover, after the insertion aid 50 has been connected to the gastrostomy catheter 10, the shaft 21 and the bending instrument 40 etc. can pass through the insertion aid 50. Furthermore, engaging parts which can be detached from each other may be provided between the reference position establishment part 31 of the device 30 for preventing excessive insertion and the reinforcing rib 57 of the insertion aid 50.

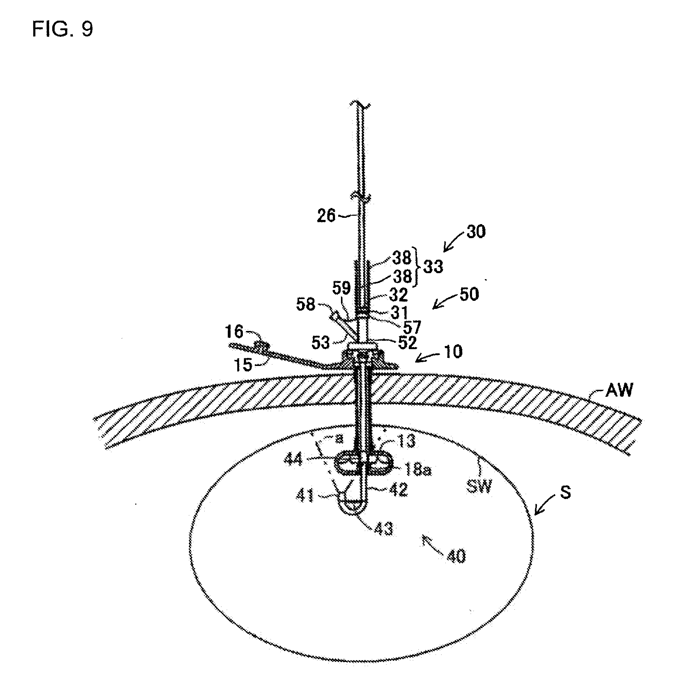

Next, air is supplied from the air supply device to inside the air supply opening 53, and the air is sent into the stomach S from the connection part 51 via the tubular part 12 of the gastrostomy catheter 10. This allows the stomach S to expand, as shown in FIG. 9. The respective areas between the gastrostomy catheter 10 and the insertion aid 50, and between the insertion aid 50 and the sheath 26 are closed off, and therefore the air inside the stomach S does not leak outside. Light which is generated by means of the light source device then passes through the wiring 25 and the light guides of the shaft 21, and is irradiated towards the stomach wall SW. Furthermore, the portion at the lower part of the shaft 21 can be made to flex together with the sheath 26 so that it is possible to change the position of irradiation of the stomach wall SW, and this is achieved by pushing the shaft 21 and the sheath 26 etc. further into the body, as shown in FIG. 9.

The mobile part 32 of the device 30 for preventing excessive insertion gradually comes closer to the reference position establishment part 31, and the position of irradiation of the stomach wall SW by the light guides moves upwards from below in accordance with this. At this time, the whole surface of the stomach wall SW can be irradiated by the light guides by turning the shaft 21 etc. in the axial direction. Then, when the mobile part 32 is in abutment with the reference position establishment part 31, the shaft 21 etc. cannot be pushed any further into the body. By means of this, the shaft 21 etc. is prevented from being inserted more than necessary into the stomach S.

In this way, by means of this medical instrument, the position of irradiation of the stomach wall SW by the light guides can be changed between the state shown in FIG. 8 in which the shaft 21, sheath 26 and bending instrument 40 extend straight ahead, and the state shown in FIG. 9 in which the shaft etc. is bent. When the shaft 21 etc. is pulled more with respect to the bending instrument 40 from the state shown in FIG. 8, the fixed part 41 of the bending instrument 40 and the portion at the lower part of the sliding part 42 pass through the through-hole 18 of the stomach-internal fixed part 13 and enter the stomach S, but the latch part 44 engages with the cylindrical engagement part 18a of the through-hole 18 and is held inside the stomach-internal fixed part 13.

Consequently, the fixed part 41 moves so as to describe the arc of a circle, with the linear linking part 43 as the radius, and the tip end portion of the shaft 21 and the sheath 26 follow this movement of the fixed part 41 while bending, and protrude inside the stomach S. The range shown by the two-dot chain line a in FIG. 9 shows the range of light irradiation by the light guides. Light which is irradiated by means of the light guides and reflected off the stomach wall SW is focused by the lens 22, after which it is sent to the image display device by way of the image guide and the wiring 24 of the shaft 21.

Images which are sent to the image display device are enlarged in the image display part of the image display device and displayed, and therefore it is possible to confirm whether or not the stomach-internal fixed part 13 of the gastrostomy catheter 10 is positioned in the correct state inside the stomach S, from the images displayed in the image display part. If it is possible to confirm that the gastrostomy catheter 10 is positioned in the correct state, an operation is carried out in which the endoscope 20 is removed from the gastrostomy catheter 10 together with the sheath 26, bending instrument 40 and insertion aid 50, and also the sheath 26, device 30 for preventing excessive insertion, the bending instrument 40 and the insertion aid 50 are removed from the shaft 21.

In this operation, the endoscope 20 is first of all pulled slightly upwards together with the sheath 26, device 30 for preventing excessive insertion, and bending instrument 40, and then in the state shown in FIG. 8, the engagement between the engagement part 55 of the insertion aid 50 and the engagement groove of the gastrostomy catheter 10 is released. At this time, the mobile part 32 of the device 30 for preventing excessive insertion gradually moves away from the reference position establishment part 31 and is positioned at the upper end of the guide holes 39 of the linking part 33. The endoscope 20 is then removed from the gastrostomy catheter 10 by pulling it upwards together with the sheath 26, device 30 for preventing excessive insertion, bending instrument 40 and insertion aid 50. In addition, the device 30 for preventing excessive insertion, bending instrument 40 and insertion aid 50 are removed from the sheath 26, after which the shaft 21 is pulled out of the sheath 26. The guide screws 36 of the mobile part 32 are loosened.

The sheath 26 and the bending instrument 40 are then disposed of, and the endoscope 20 can be reused next time. Furthermore, if the device 30 for preventing excessive insertion and the insertion aid 50 are not soiled by gastric juices or the like, they can be reused next time. At this time, the shaft 21 and the lens 22 of the endoscope 20 do not come into contact with the liquids and residues inside the patient's body and stomach S, so they are not soiled and there is no need for the most part to clean or sterilize them. Moreover, in the operation described above, the engagement between the engagement part 55 of the insertion aid 50 and the engagement groove of the gastrostomy catheter 10 is released, and the endoscope 20 is removed from the gastrostomy catheter 10 together with the bending instrument 40 and the insertion aid 50, but it is also possible to remove the endoscope 20 etc. from the insertion aid 50, and then to release the engagement between the engagement part 55 of the insertion aid 50 and the engagement groove of the gastrostomy catheter 10.

Furthermore, when nutrient fluid is supplied to the patient's stomach S, for example, by way of the gastrostomy catheter 10 which is positioned in the patient's body, a connector for a tube extending from a container housing the nutrients is connected to the insertion hole 14 of the gastrostomy catheter 10. In this state, nutrients are supplied to the patient by way of the tube and the gastrostomy catheter 10. At this time, nutrients coming out of the tubular part 12 pass from the stomach-internal fixed part 13 through each of the linking parts 13a, and enter the stomach S. Furthermore, after use, the tube from the container of nutrients is removed from the insertion hole 14 of the gastrostomy catheter 10, and the insertion hole 14 is closed using the stopper part 16. Then, when it becomes necessary to replace the gastrostomy catheter 10 after a prescribed period of use, it can be replaced with a new gastrostomy catheter 10. Here too, the position of the gastrostomy catheter 10 in the fistula can be confirmed using the endoscope 20, device 30 for preventing excessive insertion, bending instrument 40 and insertion aid 50 which have been described above.

In this way, the device 30 for preventing excessive insertion according to this embodiment consists of the reference position establishment part 31, mobile part 32, and linking part 33, and the linking part 33 can change the gap between the reference position establishment part 31 and the mobile part 32 within a range which is no greater than a set length. Consequently, when the position of the gastrostomy catheter 10 is confirmed using the endoscope 20 provided with the bending instrument 40, the tip end portion of the endoscope 20 bends suitably in a feasible range so as to be able to observe the whole of the inside of the stomach S.

In other words, when the position of the gastrostomy catheter 10 is confirmed, if the endoscope 20 to which the device 30 for preventing excessive insertion etc. is attached is inserted from the insertion hole 14, the tip end portion of the endoscope 20 gradually bends, and the mobile part 32 abuts the reference position establishment part 31 at the point where the whole of the inside of the stomach S can be observed by the endoscope 20, and the mobile part 32 cannot move any further. By this means, the endoscope 20 cannot be inserted any further either, and therefore it is possible to prevent the endoscope 20 from being inserted more than necessary into the stomach S. As a result, it is possible to prevent the bending portion of the endoscope 20 from increasing in size, which makes accurate observation impossible, and to prevent damage to the endoscope 20.

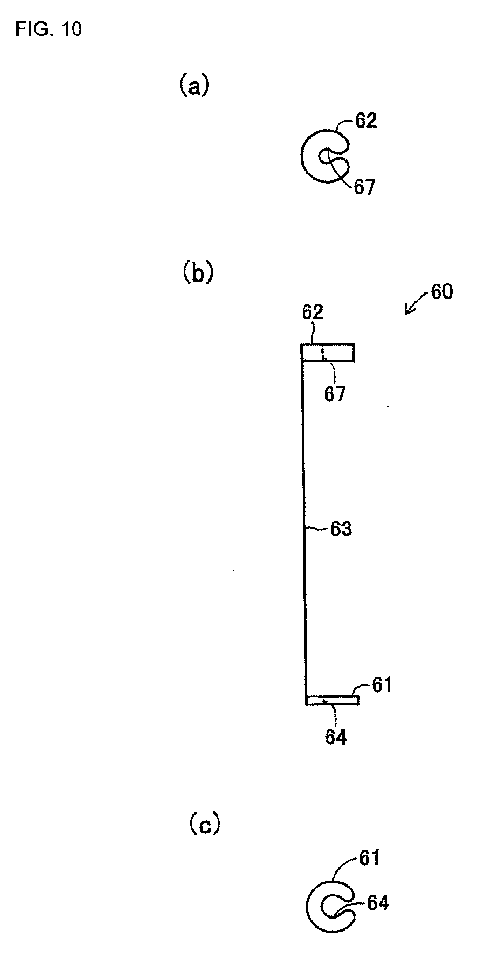

Figure 10 shows a device 60 for preventing excessive insertion according to a **second embodiment** of the present invention. The device 60 for preventing excessive insertion consists of a reference position establishment part 61, a mobile part 62 and a linking part 63. The reference position establishment part 61 consists of an elastic plate which is substantially C-shaped when viewed from above, and a through-hole 64 which is formed in the centre is of a size that enables the shaft 21 covered by the sheath 26 to pass through with clearance. The mobile part 62 consists of an elastic plate which is substantially C-shaped when viewed from above, and a through-hole 67 which is formed in the centre is of a size that enables it to fasten onto the shaft 21 covered by the sheath 26.

The linking part 63 links the reference position establishment part 61 to the mobile part 62 due to the fact that it is formed as a single piece with the reference position establishment part 61 and the mobile part 62, and it consists of a narrow, flexible linear part which runs vertically. The reference position establishment part 61 and the mobile part 62 are both flexible, and the gap between both ends can be enlarged so that the shaft 21 covered by the sheath 26 can be inserted into the respective through-holes 64, 67, by pushing the area between both ends with the outer peripheral surface of the shaft 21 covered by the sheath 26.

At this time, the reference position establishment part 61 can move in the axial direction of the shaft 21, and the mobile part 62 is fixed to the shaft 21. Consequently, the reference position establishment part 61 and the mobile part 62 can advance and retract with respect to each other within a range that is no greater than the length of the linking part 63. In other words, with the device 60 for preventing excessive insertion, the length of the linking part 63 is the set length pertaining to the present invention. The structural components of the medical instrument provided with the device 60 for preventing excessive insertion other than the device 60 for preventing excessive insertion are the same as those for the medical instrument provided with the device 30 for preventing excessive insertion, other than the device 30 for preventing excessive insertion.

When the position of the gastrostomy catheter 10 is confirmed by means of the endoscope 20 using the device 60 for preventing excessive insertion configured in the manner described above, the endoscope 20 to which are attached the sheath 26, bending instrument 40 and insertion aid 50 is first of all positioned above the gastrostomy catheter 10. The endoscope 20 and other components in this state are moved down and then insertion of the endoscope 20 etc. is stopped at the point where it has been ascertained on the image display part of the image display device that the tip end of the endoscope 20 has reached the cylindrical engagement part 18a. In this state, an operation is carried out to attach the device 60 for preventing excessive insertion to the shaft 21, with the sheath 26 interposed.

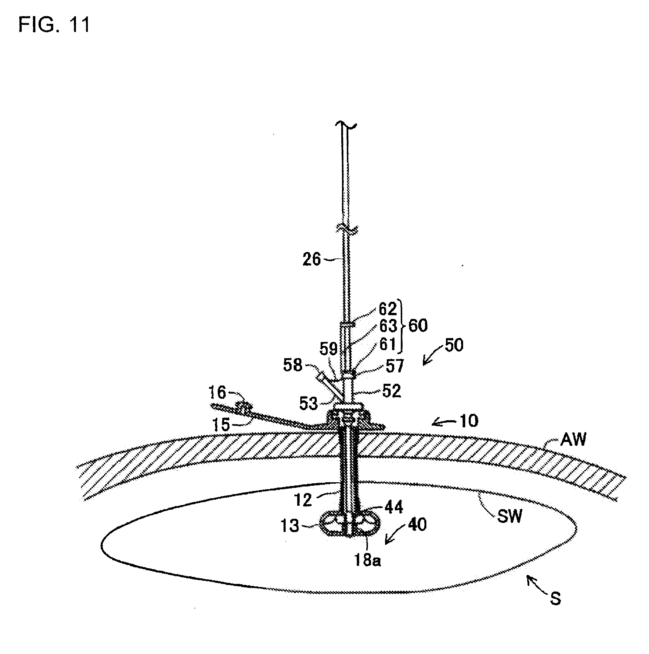

The reference position establishment part 61 is attached to the shaft 21 by first of all bringing the reference position establishment part 61 of the device 60 for preventing excessive insertion into abutment with the upper surface of the reinforcing rib 57 of the insertion aid 50 so that the area between both ends of the reference position establishment part 61 are pressed by the shaft 21. Next, with the linking part 63 extending straight ahead vertically, the mobile part 62 is fixed to the shaft 21 by pressing the area between the two end parts of the mobile part 62 with the shaft 21, and the state shown in FIG. 11 is reached. Next, the stomach S is expanded by sending air into the stomach S, after which the shaft 21 etc. is pushed further into the body.

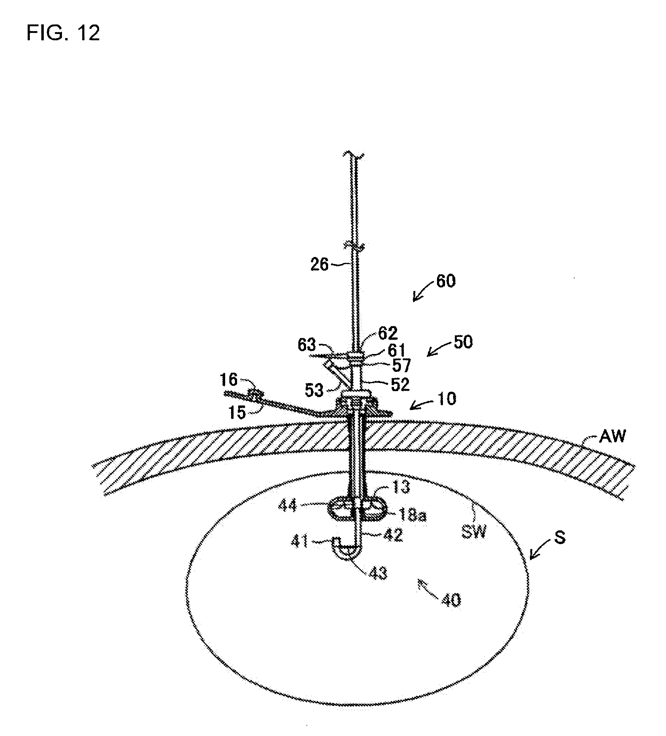

By means of this, as shown in FIG. 12, the portion at the lower part of the shaft 21 bends together with the sheath 26 so that the position of irradiation of the stomach wall SW by the light guides can be changed. Here too, the mobile part 62 gradually moves closer to the reference position establishment part 61, and the position of irradiation of the stomach wall SW by the light guides moves upwards from below in accordance with this. At this time, the linking part 63 bends and projects outwards. Here too, the whole surface of the stomach wall SW can be irradiated by the light guides by turning the shaft 21 etc. in the axial direction. Then, when the mobile part 62 is in abutment with the reference position establishment part 61, the shaft 21 etc. cannot be pushed any further into the body. By this means, the shaft 21 etc. is prevented from being inserted more than necessary into the stomach S.

According to the device 60 for preventing excessive insertion, the linking part 63 can be endowed with a simple configuration. Furthermore, when the shaft 21 is inserted into the gastrostomy catheter 10, the operation to turn the shaft in the axial direction is simple. Furthermore, when the shaft 21 is inserted into the gastrostomy catheter 10, the length of insertion of the shaft 21 is determined at the point when the mobile part 62 is fixed to the shaft 21, even if the reference position establishment part 61 is separated from the reinforcing rib 57 of the insertion aid 50, and therefore there are no difficulties unless the reference position establishment part 61 and mobile part 62 are removed from the shaft 21. The other operational effects of the device 60 for preventing excessive insertion and the medical instrument provided with the device 60 for preventing excessive insertion are the same as the device 30 for preventing excessive insertion and the medical instrument provided with the device 30 for preventing excessive insertion described above.

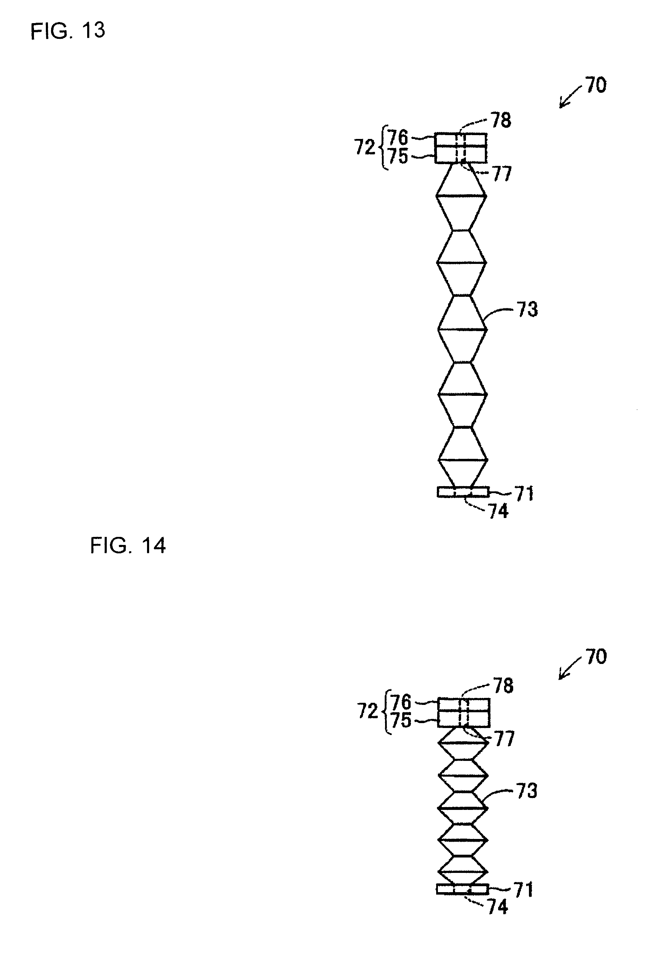

Figures 13 and 14 show a device 70 for preventing excessive insertion according to a **third embodiment** of the present invention. The device 70 for preventing excessive insertion consists of a reference position establishment part 71, a mobile part 72 and a linking part 73. The reference position establishment part 71 consists of an annular body in the centre of the disc of which is formed a through-hole 74 through which the shaft 21 covered by the sheath 26 can pass with clearance. The mobile part 72 consists of a mobile part main body 75 and a fastening screw 76 which screws onto the upper end of the mobile part main body 75. The mobile part main body 75 has a smaller diameter at its centre than the through-hole 74 of the reference position establishment part 71, but it consists of an annular body formed with a through-hole 77 through which the shaft 21 covered by the sheath 26 can pass.

A screw (not depicted) is then formed on the outer periphery at the top of the mobile part main body 75, and a plurality of flexible fastening pieces (not depicted) extending upwards are provided around the circumference at the upper-end edge of the mobile part main body 75. Furthermore, the fastening screw 76 consists of an annular body in the centre of which is formed a through-hole 78 through which the shaft 21 covered by the sheath 26 can pass, and a screw with which the screw of the mobile part main body 75 screws together is formed on the inner peripheral surface. When the screw of the fastening screw 76 is screwed together with the screw of the mobile part main body 75, the flexible fastening pieces of the mobile part main body 75 can be made to project to the inside of the mobile part main body 75 by turning the fastening screw 76 in the direction of fastening with respect to the mobile part main body 75. By this means, the mobile part 72 can be fixed to the shaft 21 covered by the sheath 26.

The linking part 73 is formed as a single piece with the reference position establishment part 71 and the mobile part main body 75 of the mobile part 72, and it consists of a substantially cylindrical bellows-like body which can extend vertically. The device 70 for preventing excessive insertion is extendible between the state shown in FIG. 13 in which the linking part 73 is extended to its maximum length, and the state shown in FIG. 14 in which the linking part 73 is contracted to its minimum length. That is to say, with the device 70 for preventing excessive insertion, the difference between the maximum length and the minimum length of the linking part 73 constitutes the set length according to the present invention. The configuration of the components of the medical instrument provided with the device 70 for preventing excessive insertion other than the device 70 for preventing excessive insertion is the same as that of the components of the medical instrument provided with the device 30 for preventing excessive insertion other than the device 30 for preventing excessive insertion described above.

When the position of the gastrostomy catheter 10 is confirmed by means of the endoscope 20 using the device 70 for preventing excessive insertion configured in the manner described above, the endoscope 20 to which are attached the sheath 26, bending instrument 40, insertion aid 50 and device 70 for preventing excessive insertion is first of all positioned above the gastrostomy catheter 10. The endoscope 20 and other components in this state are moved down, and the portion at the tip end is inserted into the insertion hole 14 of the gastrostomy catheter 10 and then insertion of the endoscope 20 etc. is stopped at the point where it has been ascertained on the image display part of the image display device that the tip end of the endoscope 20 has reached the cylindrical engagement part 18a. The operation to attach the device 70 for preventing excessive insertion to the shaft 21, with the sheath 26 interposed, is then carried out.

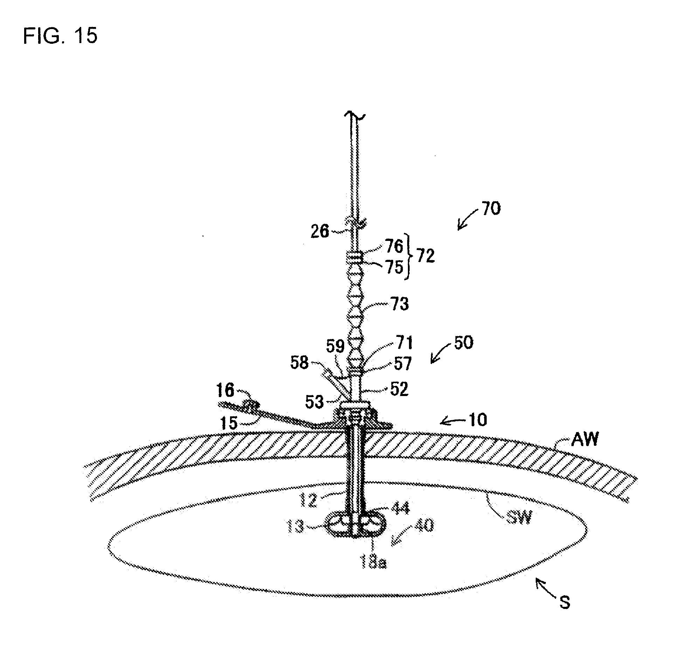

In a state in which the reference position establishment part 71 of the device 70 for preventing excessive insertion is in abutment with the upper surface of the reinforcing rib 57 of the insertion aid 50, the mobile part 72 is pulled upwards to vertically extend the linking part 73. In this state, the mobile part 72 is fixed to the shaft 21 to achieve the state shown in FIG. 15 by turning the fastening screw 76 of the mobile part 72 in the direction of fastening with respect to the mobile part main body 75. Next, the stomach S is expanded by sending air into the stomach S, after which the shaft 21 etc. is pushed further into the body.

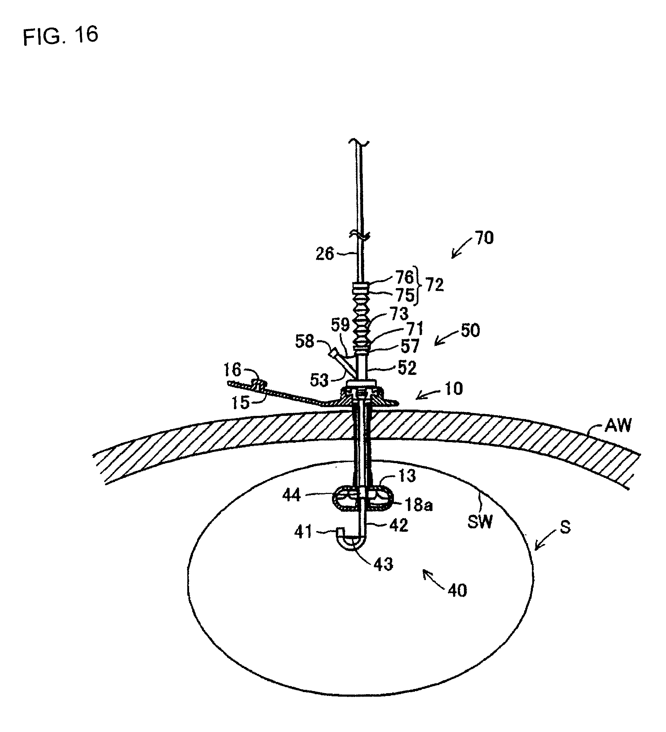

By this means, as shown in FIG. 16, the portion at the lower part of the shaft 21 bends together with the sheath 26 so that the position of irradiation of the stomach wall SW by the light guides can be changed. Here too, , the mobile part 72 gradually moves closer to the reference position establishment part 71, and the position of irradiation of the stomach wall SW by the light guides moves upwards from below in accordance with this. At this time, the linking part 73 folds and contracts. Here too, the whole surface of the stomach wall SW can be irradiated by the light guides by turning the shaft 21 etc. in the axial direction. Then, when the linking part 73 has contracted to its minimum length, the shaft 21 etc. cannot be pushed any further inside the body. By this means, the shaft 21 etc. is prevented from being inserted more than necessary into the stomach S.

According to the device 70 for preventing excessive insertion, the whole of the device 70 for preventing excessive insertion contracts along the shaft 21, and therefore the linking part 73 does not impede operation when the shaft 21 and other components are inserted into the gastrostomy catheter 10. Furthermore, the mobile part 72 can be fixed more reliably to the shaft 21. The other operational effects of the device 70 for preventing excessive insertion and the medical instrument provided with the device 70 for preventing excessive insertion are the same as the device 30 for preventing excessive insertion and the medical instrument provided with the device 30 for preventing excessive insertion described above.

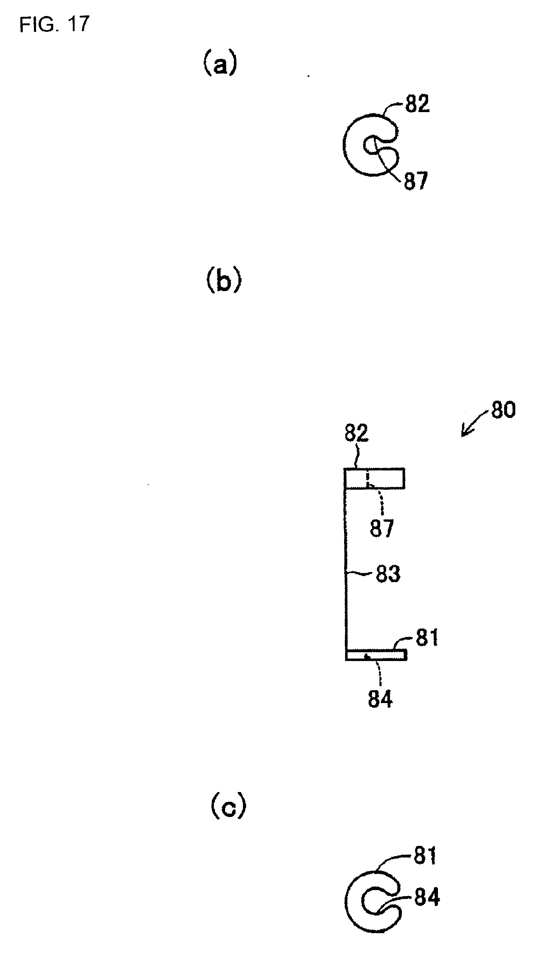

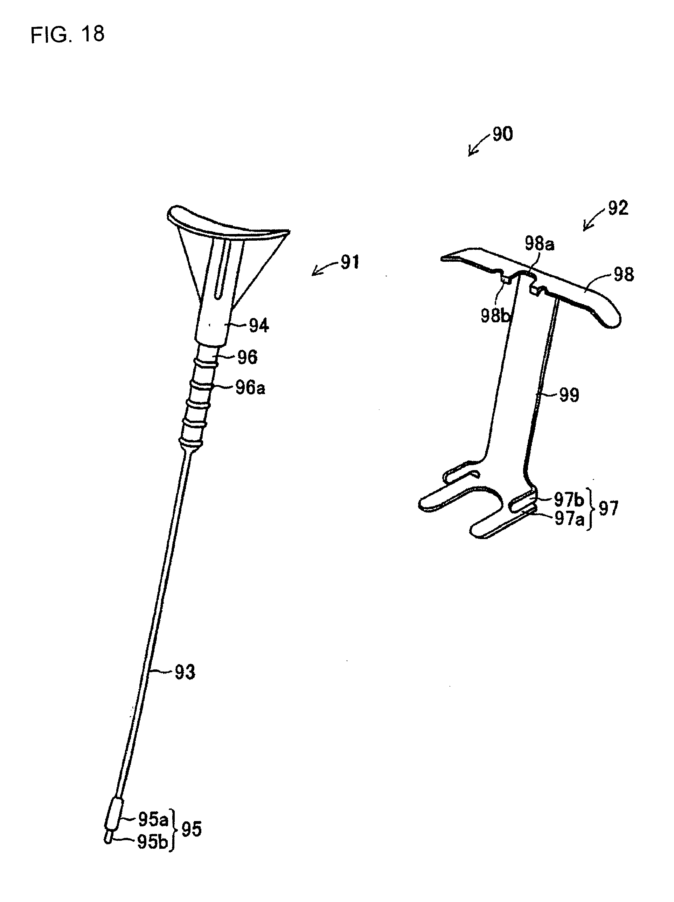

Figure 17 shows a device 80 for preventing excessive insertion according to a **fourth embodiment** of the present invention. The device 80 for preventing excessive insertion is used when the gastrostomy catheter 10 is fitted into a fistula formed in the patient's body using a fitting instrument 90 (see FIG. 18), and it consists of a reference position establishment part 81, mobile part 82 and linking part 83. The reference position establishment part 81 consists of an elastic plate which is substantially C-shaped when viewed from above, and a through-hole 84 of which one side is open is formed in the centre thereof. The mobile part 82 consists of an elastic plate which is substantially C-shaped when viewed from above, and a through-hole 87 of which one side is open is formed in the centre thereof. The through-hole 84 of the reference position establishment part 81 is larger than the insertion hole 87 of the mobile part 82.

The linking part 83 links the reference position establishment part 81 to the mobile part 82 due to the fact that it is formed as a single piece with the reference position establishment part 81 and the mobile part 82, and it consists of a narrow, flexible linear part which runs vertically. Furthermore, the reference position establishment part 81 and the mobile part 82 are both flexible, and it is possible to widen the gap between both ends, and when the force to widen the gap is released, they return to their original shape due to the elasticity. In other words, the device 80 for preventing excessive insertion is configured in the same manner as the device 60 for preventing excessive insertion described above, but with a linking part that is shorter than the linking part 63, and the reference position establishment part 81 can be attached in a state in which it can move in the longitudinal direction and the mobile part 82 can be fixed, with respect to a specific rod-like member. Consequently, the reference position establishment part 81 and the mobile part 82 can advance and retract with respect to each other within a range that is no greater than the length of the linking part 83. In other words, with the device 80 for preventing excessive insertion, the length of the linking part 83 constitutes the set length according to the present invention.

Figure 18 shows the fitting instrument 90 consisting of a push-rod 91 which acts as the rod-like member according to the present invention, and an engagement member 92 with which the push-rod 91 can engage. The push-rod 91 is provided with a main body part 93 comprising a rod body made of stainless steel, and a grip part 94 made of plastic. A pushing part 95 made of plastic is attached to the tip end of the main body part 93. The pushing part 95 consists of a fixed part 95a which is fixed to the main body part 93 in a state in which the peripheral surface of the lower end of the main body part is covered, and a pushing piece 95b which extends down from the lower end of the fixed part 95a.

The outer diameter of the fixed part 95a is set to be greater than the diameter of the through-hole 18 of the gastrostomy catheter 10, and the diameter of the pushing piece 95b is set to be smaller than the diameter of the through-hole 18. Accordingly, when the push-rod 91 is pushed downwards from the insertion hole 14 of the gastrostomy catheter 10, the pushing piece 95b enters the through-hole 18, and the fixed part 95a achieves a state in which its lower surface is in abutment with the upper surface of the cylindrical engagement part 18a. Consequently, when the push-rod 91 is pushed further downwards into the gastrostomy catheter 10, the stomach-internal fixed part 13 becomes elongate and extends.

Furthermore, a cylindrical part 96 which is configured as a single piece with the grip part 94 is formed to cover the peripheral surface of the main body part 93 at the lower portion of the grip part 94 at the top portion of the main body part 93, and a plurality of engaging steps 96a are formed on the outer peripheral surface thereof. The engaging steps 96a comprise annular protrusions which are semi-circular in cross section and are formed around the periphery of the cylindrical part 96, and five of them are formed around the axial direction of the cylindrical part 96 at constant spacing. Furthermore, the upper surface of the grip part 94 is formed with an indented arcuate curved surface so that a hand or finger, especially the thumb, can be readily pressed against it when it is operated.

The engagement member 92 is endowed with the shape shown in FIG. 18 by processing a sheet of stainless steel, and it is provided with a lower engagement part 97 and an upper engagement part 98. The lower engagement part 97 and the upper engagement part 98 are linked by means of a linking piece 99 which is a rectangular plate that is longer in the vertical direction. The lower engagement part 97 consists of: a retaining piece 97a which is like a horizontal plate and is formed to be orthogonal with the linking piece 99, extending horizontally towards the front of the drawing from the lower end of the linking piece 99; and a pair of hook parts 97b which are like vertical plates running orthogonal to the linking part 99 from both sides at the lower end portion of the linking part 99, and are provided parallel to the retaining piece 97a, constantly spaced from the retaining piece 97a.

The shape of the retaining piece 97a when viewed from above is substantially U-shaped, with the tip ends being open, and the shape of the hook parts 97b when viewed from the side is substantially rectangular, being elongate in the horizontal direction and with the tip end being semi-circular. Furthermore, the inner portion of the substantial U-shape of the retaining piece 97a is formed as a recess of a size such that the boundary part between the external fixed part 11 and the tubular part 12 of the gastrostomy catheter 10 can enter it, and the gap between the retaining piece 97a and the hook parts 97b is set to be of a size such that the projecting pieces 11b, 11c are gripped from above and below. The upper engagement part 98 is formed to be orthogonal with the linking piece 99, extending horizontally towards the front of the drawing from the upper end of the linking piece 99, and it consists of a transversely-long horizontal plate-like body which extends to both sides of the linking piece 99.

Furthermore, the length of the upper engagement part in the front to rear direction is set to be shorter, and an engaging recess 98a into which the portions between the engaging steps 96a of the push-rod 91 can be inserted is formed in the centre thereof at the front. Furthermore, a pair of protrusions 98b, for engaging with the engaging steps 96a to prevent the release of the engagement between the push-rod 91 and the engaging member 92 when a portion between the engaging steps 96a of the push-rod 91 has entered the engaging recess 98a, are provided on both sides of the engaging recess 98a at the front of the upper engagement part 98. Furthermore, the portions on both the left and right of the upper engagement part 98 are curved downwards in order to facilitate the operation to grasp them with the hand, and the tip end of the retaining piece 97a is curved downwards in order to prevent the release of the engagement with the projecting pieces 11b, 11c.

When the fitting instrument 90 and device 80 for preventing excessive insertion which are configured in this manner are used to make the gastrostomy catheter 10 positioned in the fistula formed in the patient's abdomen, the push-rod 91 is first of all inserted downwards into the tubular part 12 from the insertion hole 14 which is in an open state. The pushing piece 95b of the push-rod 91 is then pushed into the through-hole 18 of the stomach-internal fixed part 13, and the fixed part 95a is brought into abutment with the upper surface of the cylindrical engagement part 18a. Next, the projecting pieces 11b, 11c of the gastrostomy catheter 10 in this state are grasped by the retaining piece 97a and hook parts 97b of the engagement member 92, along with which the engagement member 92 is attached to the gastrostomy catheter 10 and the push-rod 91, in a state in which the main body part 93 is positioned inside the engaging recess 98a.

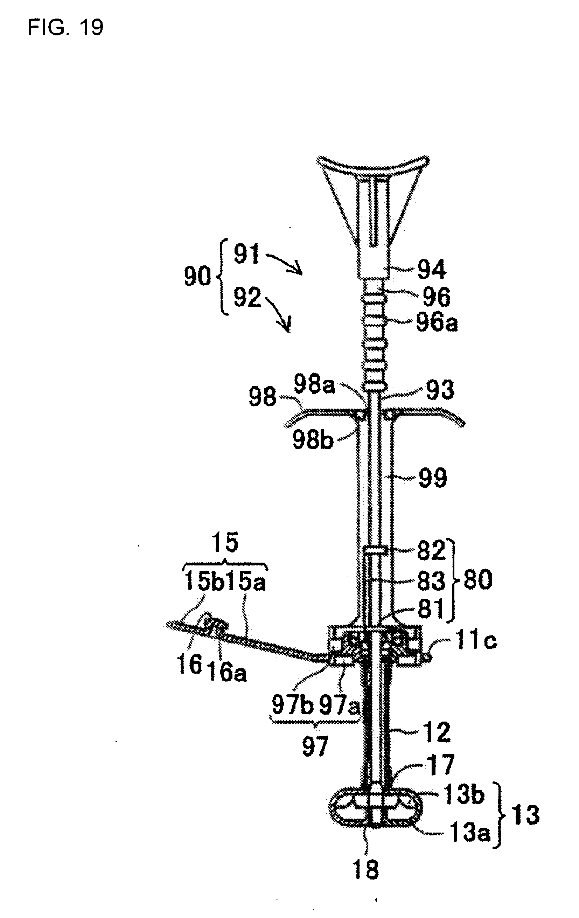

An operation is then carried out to attach the device 80 for preventing excessive insertion to the push-rod 91. The reference position establishment part 81 of the device 80 for preventing excessive insertion is attached to the main body part 93 by bringing the reference position establishment part 81 into abutment with the upper surface of the external fixed part 11 of the gastrostomy catheter 10, so that the area between both ends of the reference position establishment part 81 is pushed by the main body part 93. Next, the mobile part 82 is fixed to the main body part 93 by pushing the area between both ends of the mobile part 82 with the main body part 93, in a state in which the linking part 83 is extended vertically in a straight line, and the state shown in FIG. 19 is achieved. In this state, the push rod 91 is pushed further inside the gastrostomy catheter 10.

With the upper surface of the grip part 94 being pushed using the hand, and the pushing piece 95b being prevented from separating from the through-hole 18, the fingers are hooked under the upper engagement part 98 and the engagement member 92 is pulled upwards. By this means, the stomach-internal fixed part 13 is extended to make it elongate, and the mobile part 82 is gradually brought closer to the reference position establishment part 81. At this time, the linking part 83 is bent so that it protrudes outwards. When the mobile part 82 is in abutment with the reference position establishment part 81, the push-rod 91 cannot be pushed any further into the gastrostomy catheter 10. This means that the stomach-internal fixed part 13 is prevented from extending more than necessary, and the stomach-internal fixed part 13 becomes appropriately narrow to pass through the fistula.

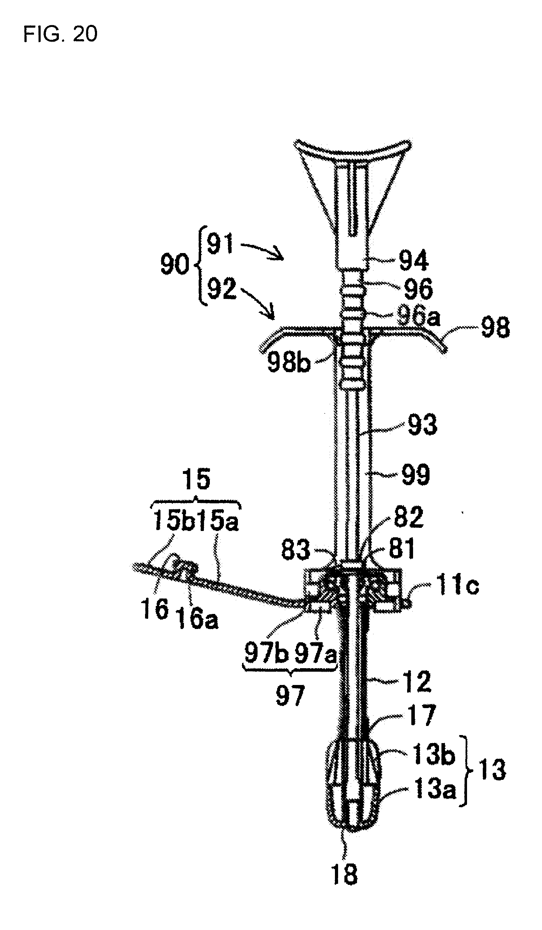

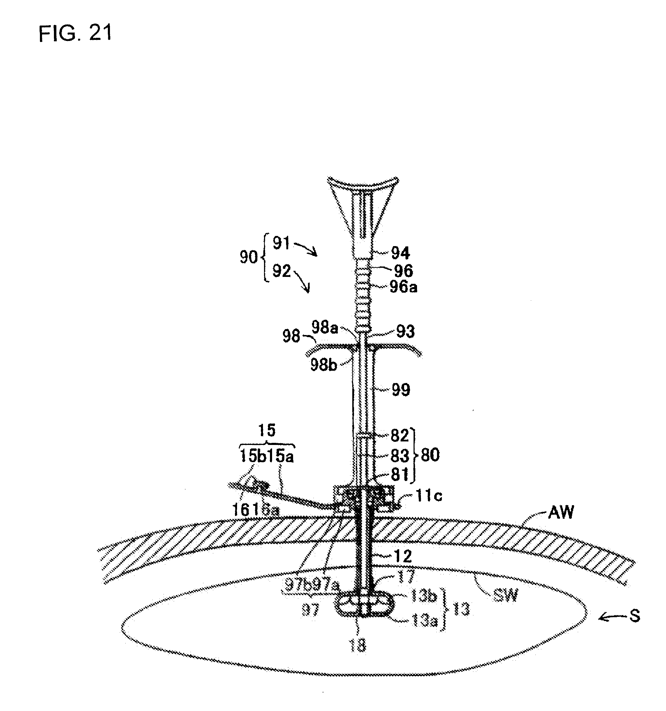

In this state, the edge of the engaging recess 98a of the engagement member 92 is engaged with a prescribed opposing engaging step 96a of the push-rod 91. By means of this, as shown in FIG. 20, the tubular part 12 and the stomach-internal fixed part 13 reach a state in which they approximate a single rod. The stomach-internal fixed part which has extended in this way passes through the fistula formed in the abdominal wall AW and stomach wall SW of the patient together with the tip end portion of the push-rod 91. Then, once the stomach-internal fixed part has entered the patient's stomach S, the fingers are hooked under the upper engagement part 98 to pull the engagement member 92 upwards, and the engaging recess 98a is released from the engaging step 96a. By this means, as shown in FIG. 21, the shape of the stomach-external fixed part 13 reverts to its original substantially spherical, expanded state, and the push-rod 91 is moved upwards. Consequently, the mobile part 82 also moves upwards together with the push-rod 91, and the linking part 83 reaches a state in which it extends straight.

As a result, the gastrostomy catheter 10 is maintained in a state in which it is prevented from being withdrawn from the fistula and is fitted in the patient's abdomen. In this state, the retaining piece 97a and the hook parts 97b are removed from the projecting pieces 11b, 11c, and the engagement member 92 is removed from the gastrostomy catheter 10, and also the push-rod 91 is withdrawn from the gastrostomy catheter 10. The strip-shaped linking part 15a is then bent and the stopper part 16 is pushed into the insertion hole 14 to close off the insertion hole 14. Furthermore, the operation described for the first embodiment is carried out when a patient is to be supplied with fluids such as nutrients and food in fluid form.

Then, when the gastrostomy catheter 10 becomes stretched or undergoes some other physical change after a certain period of use so that it has to be replaced, the operation described above is carried out to make the stomach-internal fixed part elongate, after which an operation is carried out to withdraw it from the fistula, but in this instance the device for preventing excessive insertion may or may not be used. With the device 80 for preventing excessive insertion according to this mode of embodiment, the stomach-internal fixed part 13 of the gastrostomy catheter 10 can be extended to make it appropriately narrow, and therefore the operation to make the gastrostomy catheter 10 positioned in the fistula is simple.

The present invention has been devised in order to deal with one or more of the problems described above, and it aims to provide a device for preventing excessive insertion rod-like member into a gastrostomy catheter, and a medical instrument is provided with the same.

In order to achieve the aim mentioned in the preceding paragraph, structural features of the device are designed based on the fact that it is used for preventing excessive insertion which is used when a rod-like member is inserted into a gastrostomy catheter. The catheter comprises in one embodiment a tubular part and a stomach-internal fixed part comprising an expanded body which links with the tip end of the tubular part and is provided with a through-hole at the tip end, the stomach-internal fixed part extending in an elongate fashion due to a portion on the through-hole side being pushed in the direction of the tip end side. The device comprises a reference position establishment part which can be installed at a prescribed portion on the base end opening side of the gastrostomy catheter in a state in which it can move in the longitudinal direction of the rod-like member; a mobile part which can be fixed to a prescribed portion of the rod-like member; and a linking part which can change the gap between the reference position establishment part and the mobile part within a range which is no greater than a set length, and further links the reference position establishment part and the mobile part.

The device for preventing excessive insertion according to one embodiment of the present invention comprises a reference position establishment part, a mobile part, and a linking part for linking the reference position establishment part and the mobile part. The linking part can change the gap between the reference position establishment part and the mobile part within a range which is no greater than a set length. The set length is the most suitable length or the maximum length when the rod-like member is inserted into the gastrostomy catheter. Then, when the rod-like member is inserted into the gastrostomy catheter, the tip end of the rod-like member is first inserted from the base end opening, and positioned at a prescribed position in the vicinity of the through-hole of the stomach-internal fixed part. In this state, the reference position establishment part is placed on the base end opening side, and also the operating part is fixed to the rod-like member in a state in which the length by which the mobile part can move is the set length.

Then, when the rod-like member is further inserted into the tip end side of the gastrostomy catheter, the reference position establishment part maintains a state in which it is placed at a portion on the base end opening side of the gastrostomy catheter. The rod-like member is inserted therein, and the mobile part moves to the reference position establishment part side together with the rod-like member. Consequently, when the gap between the reference position establishment part and the mobile part has become smaller and the movement distance of the mobile part has reached the set length, the mobile part can move no further, and therefore the rod-like member to which the mobile part is fixed cannot be inserted any further either. The insertion length of the rod-like member into the gastrostomy catheter accurately reaches the set length. Moreover, the reference position establishment part may be directly placed at the base end opening of the gastrostomy catheter, or it may be placed with another member interposed.

Furthermore, the reference position establishment part may be engaged with the base end opening of the gastrostomy catheter, or with another member attached to the base end opening, or it may simply be in a state of abutment with the base end opening of the gastrostomy catheter or the other member. In addition, a component provided with an external fixed part linked to the base end of the tubular part and formed with a tubular insertion hole passing through inside may be used as the gastrostomy catheter, and the reference position establishment part may be placed directly on the external fixed part, or it may be placed with another member interposed. If the reference position establishment part is placed directly on the external fixed part, it is simple to place the reference position establishment part on the gastrostomy catheter.

Furthermore, other structural features of the device for preventing excessive insertion according to an embodiment of the present invention describe the reference position establishment part as consisting of an annular body inside which the rod-like member can be inserted. Hence, the reference position establishment part can be attached to the rod-like member simply by inserting the rod-like member into the reference position establishment part, and therefore the operation to attach the reference position establishment part is simple. In such an embodiment, the reference position establishment part may be formed as a cylindrical shape, or it may be formed as an annular shape.

Furthermore, additional structural features of the device for preventing excessive insertion according to an embodiment of the present invention describe the reference position establishment part as consisting of an elastic member which is substantially C-shaped in transverse section, and the rod-like member can be inserted into the reference position establishment part in a state in which the gap between the end parts of the substantial C-shape of the elastic member is widened. Hence, the reference position establishment part can be attached to the rod-like member in a state in which the tip end of the rod-like member has been inserted up to the stomach-internal fixed part of the gastrostomy catheter, and therefore the operation when the rod-like member is inserted into the gastrostomy catheter is simple.

Furthermore, additional structural features of the device for preventing excessive insertion according to an embodiment of the present invention describe the mobile part as comprising an annular body provided with a fastening mechanism, and the mobile part can be fixed to a prescribed portion of the rod-like member by fastening the rod-like member using the fastening mechanism in a state in which the rod-like member is inserted into the mobile part. Hence, the mobile part can be easily attached to the rod-like member simply by inserting the rod-like member into the mobile part, and therefore the operation to attach the mobile part is simple. Furthermore, it is possible to use, as the fastening mechanism, a mechanism in which a cylindrical body, which is formed with a plurality of fastening pieces on the edge of the opening, and an annular body are screwed together. The fastening pieces are urged inwards by means of the screwing process, or by any mechanism with which it is possible to cause a screw to project inwards from the outer peripheral surface by screwing it into a screw hole passing from the outer peripheral surface to the inner peripheral surface of the annular body.

Furthermore, additional structural features of the device for preventing excessive insertion according to an embodiment of the present invention describe the mobile part as comprising an elastic member which can pinch the outer peripheral part of the rod-like member from both sides. The mobile part can be fixed to a prescribed portion of the rod-like member by first inserting the rod-like member into the mobile part in a state in which the mobile part is widened, and then fastening the rod-like member using the elasticity of the mobile part.

Hence, the mobile part can be attached to the rod-like member in a state in which the tip end of the rod-like member has been inserted up to the stomach-internal fixed part of the gastrostomy catheter, and therefore the operation when the rod-like member is inserted into the gastrostomy catheter is simple. Moreover, the mobile part may be a component in which the mobile part itself is formed from an elastic material, or it may be a component which can fix the rod-like member by pressing a certain piece against it with an elastic member such as a spring interposed. If the mobile part comprises an elastic material, it is possible to use a member having a substantially C-shaped transverse section, and the assembly can be designed so that the rod-like member can be inserted into the mobile part in a state in which the end parts of the substantial C-shape of the mobile part are widened, and the mobile part can be fixed to a prescribed portion of the rod-like member by fastening using the elasticity of the mobile part. Furthermore, if an elastic member such as a spring is used in the mobile part, a clip or other member like a clothes peg may be used.

Furthermore, additional structural features of the device for preventing excessive insertion according to an embodiment of the present invention describe the linking part as consisting of a rigid rail member, and the mobile part can slide on the linking part which comprises the rail member. Hence, the mobile part can be accurately moved by the set length.

Furthermore, additional structural features of the device for preventing excessive insertion according to an embodiment of the present invention describe the linking part as consisting of a flexible linear part which links the respective prescribed portions of the reference position establishment part and of the mobile part. This means that the linking part can be simply configured. Furthermore, when the rod-like member is inserted into the gastrostomy catheter, the mobile part can be accurately moved by the set length even if the rod-like member rotates about the axis of the gastrostomy catheter.

Furthermore, additional structural features of the device for preventing excessive insertion according to an embodiment of the present invention lie in the fact that the linking part may comprise an extendible bellows-like part inside which the rod-like member can be inserted. Hence, the overall device for preventing excessive insertion is extendible along the rod-like member, and therefore the linking part does not impede operation when the rod-like member is inserted into the gastrostomy catheter.

Furthermore, structural features of the medical instrument according to an embodiment of the present invention describe that the medical instrument is provided with the device for preventing excessive insertion, where the medical instrument comprises an endoscope provided with a mechanism for bending the tip end portion when the rod-like member is inserted into the gastrostomy catheter and the tip end portion projects outward by a prescribed length from the through-hole of the stomach-internal fixed part. Hence, the tip end portion of the endoscope can be bent to a suitable angle inside the stomach, and therefore it is possible to reliably confirm the state of the gastrostomy catheter and the state of the stomach wall. The set length is set at the minimum length in the range whereby the whole surface of the stomach wall can be checked while the tip end portion of the endoscope is changed from a linear state to a bent state. In other words, the set length is set at a length such that any insertion beyond this is unnecessary.

Furthermore, other structural features of the medical instrument according to an embodiment of the present invention describe that the medical instrument is provided with the device for preventing excessive insertion, where the medical instrument comprises a push-rod which can push a portion on the through-hole side in the direction of the tip end side in a state in which the rod-like member is inserted into the gastrostomy catheter and the tip end is engaged with the peripheral edge of the through-hole of the stomach-internal fixed part. Hence, the stomach-internal fixed part of the catheter can extend to become suitably narrow, and therefore the operation to position the gastrostomy catheter in the fistula is simple. The set length is a suitable length so that the stomach-internal fixed part can extend to a suitable thickness.

Furthermore, the device for preventing excessive insertion according to the present invention is not limited to the modes of embodiment described above, and various modifications can be made within the technical scope of the present invention. For example, in the various embodiments described above, use is made of the gastrostomy catheter 10 provided with the external fixed part 11, but in modes of embodiment 1 to 3, a gastrostomy catheter which is not provided with an external fixed part 11 may be used. The insertion aid 50 may also be omitted. In addition, the assemblies comprising the reference position establishment parts 31, 61, 71, 81, mobile parts 32, 62, 72, 82, and linking parts 33, 63, 73, 83 in the devices 30, 60, 70, 80 for preventing excessive insertion which were used in the exemplary embodiments described above can be changed, respectively. In addition, an assembly in which specific pieces are pushed into contact with the endoscope 20 or push-rod 91, with an elastic member such as a spring interposed, may also be used.

Having described the invention in detail, it will be apparent that modifications and variations are possible without departing from the scope of the invention defined in the appended claims.

When introducing elements of the present invention or the preferred embodiments(s) thereof, the articles "a", "an", "the" and "said" are intended to mean that there are one or more of the elements. The terms "comprising", "including" and "having" are intended to be inclusive and mean that there may be additional elements other than the listed elements.

In view of the above, it will be seen that the several objects of the invention are achieved and other advantageous results attained.

As various changes could be made in the above constructions without departing from the scope of the invention, it is intended that all matter contained in the above description and shown in the accompanying drawing[s] shall be interpreted as illustrative and not in a limiting sense.

## Claims

1. A device for preventing excessive insertion when a rod-like member is inserted into a gastrostomy catheter, said gastrostomy catheter comprising a tubular part and a stomach-internal fixed part, said fixed part comprising an expanded body which links with a tip end of the tubular part, said tip end provided with a through-hole, said fixed part extending in an elongate fashion due to a portion on a through-hole side being pushed in the direction of a tip end side, said device comprising:
a reference position establishment part which can be installed at a prescribed portion on a base end opening side of the gastrostomy catheter in a state in which it can move in a longitudinal direction of the rod-like member;
a mobile part which can be fixed to a prescribed portion of the rod-like member; and
a linking part which can change a gap between the reference position establishment part and the mobile part within a range which is no greater than a set length, and links the reference position establishment part and the mobile part.

2. The device of claim 1, wherein the reference position establishment part comprises an annular body inside which the rod-like member can be inserted.

3. The device of claim 1, wherein the reference position establishment part comprises an elastic member which is substantially C-shaped in transverse section, and wherein the rod-like member can be inserted into the reference position establishment part in a state in which a gap between the end parts of the substantially C-shaped elastic member is widened.

4. The device of any of claims 1 to 3, wherein the mobile part comprises an annular body provided with a fastening mechanism, and wherein the mobile part can be fixed to a prescribed portion of the rod-like member by fastening the rod-like member using the fastening mechanism in a state in which the rod-like member is inserted into the mobile part.

5. The device of any of claims 1 to 3, wherein the mobile part comprises an elastic member which can pinch a outer peripheral part of the rod-like member from both sides, and wherein the mobile part can be fixed to a prescribed portion of the rod-like member by inserting the rod-like member into the mobile part in a state in which the mobile part is widened, and fastening the rod-like member using the elasticity of the mobile part.

6. The device of any of claims 1 to 5, wherein the linking part comprises a rigid rail member, and wherein the mobile part can slide on the rigid rail member.

7. The device of any of claims 1 to 5, wherein the linking part comprises a flexible linear part, said flexible linear part linking respective prescribed portions of the reference position establishment part and of the mobile part.

8. The device of any of claims 1 to 5, wherein the linking part comprises an extendible bellows-like part, and wherein the rod-like member can be inserted into the extendible bellows-like part.

9. A medical instrument provided with the device of any of claims 1 to 8, said medical instrument comprising an endoscope provided with a mechanism for bending a tip end portion when the rod-like member is inserted into the gastrostomy catheter and the tip end projects outwards by a prescribed length from the through-hole of the stomach-internal fixed part.

10. A Medical instrument provided with the device of any of claims 1 to 8, said medical instrument comprising a push-rod, said push-rod capable of pushing a portion on the through-hole side in the direction of the tip end side in a state in which the rod-like member is inserted into the gastrostomy catheter and the tip end is engaged with the peripheral edge of the through-hole of the stomach-internal fixed part.
